(19)

Europäisches Patentamt
European Patent Office
Office européen des brevets

(11) **EP 4 112 720 A1**

(12) **EUROPEAN PATENT APPLICATION**
published in accordance with Art. 153(4) EPC

(43) Date of publication:
**04.01.2023 Bulletin 2023/01**

(21) Application number: **21761074.0**

(22) Date of filing: **01.03.2021**

(51) International Patent Classification (IPC):
*C12N 5/10* (2006.01)      *A61K 35/19* (2015.01)
*A61P 7/04* (2006.01)      *C12N 5/078* (2010.01)
*C12N 15/09* (2006.01)

(52) Cooperative Patent Classification (CPC):
**A61K 35/19; A61P 7/04; C12N 5/06; C12N 5/10;
C12N 15/09**

(86) International application number:
**PCT/JP2021/007669**

(87) International publication number:
**WO 2021/172583 (02.09.2021 Gazette 2021/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.02.2020 JP 2020034255**

(71) Applicants:
• **Otsuka Pharmaceutical Co., Ltd.
Tokyo 101-8535 (JP)**
• **Kyoto University
Kyoto-shi, Kyoto 606-8501 (JP)**

(72) Inventors:
• **HARADA Yasuo
Osaka-shi, Osaka 540-0021 (JP)**

• **HOTTA Akitsu
Kyoto-shi, Kyoto 606-8501 (JP)**
• **XU Huaigeng
Kyoto-shi, Kyoto 606-8501 (JP)**
• **SUGIMOTO Naoshi
Kyoto-shi, Kyoto 606-8501 (JP)**
• **ETO Koji
Kyoto-shi, Kyoto 606-8501 (JP)**

(74) Representative: **Hoffmann Eitle
Patent- und Rechtsanwälte PartmbB
Arabellastraße 30
81925 München (DE)**

Remarks:
The complete document including Reference
Table(s) and the Sequence Listing(s) can be
downloaded from the EPO website

(54) **GENETICALLY MODIFIED MEGAKARYOCYTE, MODIFIED PLATELET, AND METHODS RESPECTIVELY FOR PRODUCING SAID GENETICALLY MODIFIED MEGAKARYOCYTE AND SAID MODIFIED PLATELET**

(57)      A production method for a genetically modified megakaryocyte, the method including a step of introducing a CRISPR-associated (Cas) family protein and guide RNA (gRNA) into a megakaryocyte to modify a subject gene, in which the Cas family protein and the gRNA to be introduced into the megakaryocyte in the step form a complex beforehand. This production method is useful as a technique for producing a genetically modified megakaryocyte and a modified platelet.

EP 4 112 720 A1

**Description**

Field of the Invention

The present invention relates to a genetically modified megakaryocyte, a modified platelet, and methods for producing thereof.

Priority is claimed on Japanese Patent Application No. 2020-034255, filed February 28, 2020, the content of which is incorporated herein by reference.

Description of Related Art

Platelets are a type of cells produced from the cytoplasm of megakaryocytes in the bone marrow. Platelets play a central role in the formation of blood clots, and have the function of aggregating to close wounds and thereby stopping bleeding when a blood vessel wall is damaged.

Platelet transfusion is performed for the purpose of stopping bleeding or preventing bleeding by supplementing platelet components for a pathological condition in which severe bleeding is caused or bleeding is predicted due to a decrease in the number of platelets or an abnormality in the function thereof.

However, in patients with platelet refractoriness, it is known that transplanted platelets are disrupted as a result of abnormal production of antibodies against Human Leukocyte Antigen (HLA) class 1. On the other hand, when HLA-deficient platelets can be produced, it is expected that transplantation will become possible in patients with platelet refractoriness who have antibodies against HLA class 1.

In recent years, a CRISPR-Cas9 system, which is one of Clustered Regularly Interspaced Short Palindromic Repeat (CRISPR) systems that are acquired immune systems for prokaryotes, has been widely used for genome editing techniques such as gene disruption (knockout). This is a technique in which double-stranded DNA break (DSB) is induced at a desired site of genomic DNA by the above system, and thereafter a deletion or insertion is caused by the repair mechanism inherent in a host cell. Furthermore, it is known that Cas family proteins other than Cas9 can be utilized for genome editing.

As a production method for HLA protein-deficient platelets, because platelets do not have a genome, it is conceivable to disrupt an HLA gene by genome editing in megakaryocytes of the previous stage to obtain the above-mentioned platelets from HLA protein-deficient megakaryocytes. However, as disclosed in Non-Patent Literature 1 for example, it is conventionally known that it is difficult to express a foreign gene in megakaryocytes by a method other than a viral vector method. Megakaryocytes are giant cells in which the diameter ranges from 35 to 160 $\mu$m, and a part of the cell membrane is extended and torn off under intense shear stress due to turbulent flow, thereby producing platelets. That is, it is thought that the cell membrane of megakaryocytes is more flexible and tougher than other cells, and the specificity of the membrane structure makes it difficult to introduce genes from the outside.

In addition, Non-Patent Literature 2 discloses that genome editing could not be performed on megakaryocytes differentiation-induced from iPS cells. Therefore, Non-Patent Literature 2 discloses that megakaryocytes were reprogrammed again to return to iPS cells, genome editing was performed, and thereafter differentiation induction into megakaryocytes was performed again.

In addition, Non-Patent Literature 3 discloses that genome editing of megakaryocytes was performed by introducing Cas9 and gRNA into megakaryocytes using a lentiviral vector.

Citation List

[Non-Patent Literature]

[Non-Patent Literature 1]
Isakari Y., et al., Efficient gene expression in megakaryocytic cell line using nucleofection, Int J Pharm., 338 (1-2), 157-64, 2007.
[Non-Patent Literature 2]
Seo H., et al., A betal-tubulin-based megakaryocyte maturation reporter system identifies novel drugs that promote platelet production, Blood Adv., 2 (17), 2262-2272, 2018.
[Non-Patent Literature 3]
Kahr W.H., et al., Loss of the Arp2/3 complex component ARPC1B causes platelet abnormalities and predisposes to inflammatory disease, Nat Commun., 8:14816, 2017.

Summary of the Invention

Technical Problem

However, in the method of Non-Patent Literature 2, a lot of labor and time are required in inducing reprogramming into iPS cells and differentiating into megakaryocytes again, which is inefficient. Furthermore, in the method of Non-Patent Literature 3, a transgene is inserted into the genomic DNA of megakaryocytes and cannot be removed, and there is a risk that the expression of a foreign gene becomes a new antigen or that the function of the endogenous gene is adversely affected. More specifically, a Cas9 gene, sgRNA, and the like are inserted into the genome in the genome editing using the lentiviral vector. Therefore, an off-target mutagenesis risk, antigenicity by Cas9 protein expression, and an influence on the function of the endogenous gene of the genome (activation of carcinogenic genes, and the like) can be expected in the obtained megakaryocytes, making the megakaryocytes unsuitable for clinical application. Furthermore, regarding the use of lentiviruses, there are problems of limitations at implementation facilities, and high production cost, and other simpler methods have been sought.

With the foregoing background, an object of the present invention is to provide a technique for producing a genetically modified megakaryocyte and a modified platelet.

Solution to Problem

The present invention includes the following aspects.

[1] A production method for a genetically modified megakaryocyte, the method including: a step of introducing a CRISPR-associated (Cas) family protein and guide RNA (gRNA) into a megakaryocyte to modify a subject gene, in which the Cas family protein and the gRNA to be introduced into the megakaryocyte in the step form a complex beforehand.
[2] The production method according to [1], in which the introduction of the Cas family protein and the gRNA is performed by a lipofection method which is suitable for protein introduction or an electroporation method.
[3] The production method according to [1] or [2], in which the introduction of the Cas family protein and the gRNA is performed by an electroporation method.
[4] The production method according to any one of [1] to [3], in which the subject gene is (i) Human Leukocyte Antigen (HLA)-A, HLA-B, and HLA-C genes, or (ii) β2-Microglobulin (B2M) gene, and the modification is disruption of the subject gene.
[5] The production method according to any one of [1] to [4], in which the megakaryocyte is obtained by differentiating pluripotent stem cells.
[6] A genetically modified megakaryocyte in which (i) HLA-A, HLA-B, and HLA-C genes, or (ii) B2M gene is disrupted.
[7] A production method for a modified platelet, the method including: a step of obtaining a genetically modified megakaryocyte by introducing a Cas family protein and gRNA into a megakaryocyte to modify a subject gene; and a step of obtaining a modified platelet by producing a platelet from the genetically modified megakaryocyte, in which the Cas family protein and the gRNA to be introduced into the megakaryocyte in the step of obtaining a genetically modified megakaryocyte form a complex beforehand.
[8] The production method according to [7], in which the introduction of the Cas family protein and the gRNA is performed by a lipofection method which is suitable for protein introduction or an electroporation method.
[9] The production method according to [7] or [8], in which the introduction of the Cas family protein and the gRNA is performed by an electroporation method.
[10] The production method according to any one of [7] to [9], in which the megakaryocyte is obtained by differentiating pluripotent stem cells.
[11] The production method according to any one of [7] to [10], in which the subject gene is (i) HLA-A, HLA-B, and HLA-C genes, or (ii) B2M gene, and the modification is disruption of the subject gene.
[12] An HLA-deficient platelet which is deficient in an HLA-A protein, an HLA-B protein, and an HLA-C protein.
[13] The HLA-deficient platelet according to [12], which is produced by the production method according to [11].
[14] A production method for a genetically modified megakaryocyte, the method including: a step of introducing a Cas family protein and gRNA into a megakaryocyte to modify a subject gene, in which the introduction of the Cas family protein and the gRNA in the step is performed by an electroporation method.
[15] The production method according to [14], in which the megakaryocyte is obtained by differentiating pluripotent stem cells.
[16] The production method according to [14] or [15], in which the electroporation method is performed using 0.1 to 1 pmol of the Cas family protein and 0.1 to 1 pmol of the gRNA per 1000 megakaryocyte cells.
[17] The production method according to any one of [14] to [16], in which the subject gene is (i) Human Leukocyte Antigen (HLA)-A, HLA-B, and HLA-C genes, or (ii) β2-Microglobulin (B2M) gene, and the modification is disruption of the subject gene.

[18] A genetically modified megakaryocyte which is produced by the production method according to [17], in which (i) HLA-A, HLA-B, and HLA-C genes, or (ii) B2M gene is disrupted.

[19] A production method for a modified platelet, the method including: a step of obtaining a genetically modified megakaryocyte by introducing a Cas family protein and gRNA into a megakaryocyte using an electroporation method to modify a subject gene; and a step of obtaining a modified platelet by producing a platelet from the genetically modified megakaryocyte.

The present invention can also be said to include the following aspects.

[P1] A production method for a genetically modified megakaryocyte, the method including: a step of introducing a CRISPR-associated (Cas) family protein and guide RNA (gRNA) into a megakaryocyte to modify a subject gene.

[P2] The production method for a genetically modified megakaryocyte according to [P1], in which the introduction of the Cas family protein and the gRNA is performed by an electroporation method.

[P3] The production method for a genetically modified megakaryocyte according to [P1] or [P2], in which the subject gene is (i) Human Leukocyte Antigen (HLA)-A, HLA-B, and HLA-C genes, or (ii) β2-Microglobulin (B2M) gene, and the modification is disruption of the subject gene.

[P4] The production method for a genetically modified megakaryocyte according to any one of [P1] to [P3], in which the megakaryocyte is obtained by differentiating pluripotent stem cells.

[P5] A genetically modified megakaryocyte in which (i) HLA-A, HLA-B, and HLA-C genes, or (ii) B2M gene is disrupted.

[P6] A production method for a modified platelet, the method including: a step of obtaining a genetically modified megakaryocyte by introducing a Cas family protein and gRNA into a megakaryocyte to modify a subject gene; and a step of obtaining a modified platelet by producing a platelet from the genetically modified megakaryocyte.

[P7] The production method for a modified platelet according to [P6], in which the introduction of the Cas family protein and the gRNA is performed by an electroporation method.

[P8] The production method for a modified platelet according to [P6] or [P7], in which the subject gene is (i) HLA-A, HLA-B, and HLA-C genes, or (ii) B2M gene, and the modification is disruption of the subject gene.

[P9] The production method for a modified platelet according to any one of [P6] to [P8], in which the megakaryocyte is obtained by differentiating pluripotent stem cells.

[P10] An HLA-deficient platelet which is deficient in an HLA-A protein, an HLA-B protein, and an HLA-C protein.

Advantageous Effects of Invention

According to the present invention, a technique for producing a genetically modified megakaryocyte and a modified platelet can be provided.

Brief Description of the Drawings

Fig. 1 shows graphs representing the results of flow cytometry analysis in Experimental Example 1.
Fig. 2 shows graphs representing the results of flow cytometry analysis in Experimental Example 2.
Fig. 3A is a diagram representing a gating setting for flow cytometry analysis in Experimental Example 3.
Fig. 3B shows graphs representing the results of flow cytometry analysis in Experimental Example 3.
Fig. 4A shows graphs representing the results of flow cytometry analysis in Experimental Example 4.
Fig. 4B shows graphs representing the results of flow cytometry analysis in Experimental Example 4.
Fig. 5A shows graphs representing the results of flow cytometry analysis in Experimental Example 5.
Fig. 5B shows graphs representing the results of flow cytometry analysis in Experimental Example 5.
Fig. 6A shows graphs representing the results of flow cytometry analysis in Experimental Example 5.
Fig. 6B shows graphs representing the results of flow cytometry analysis in Experimental Example 5.
Fig. 7A shows graphs representing the results of flow cytometry analysis in Experimental Example 5.
Fig. 7B shows graphs representing the results of flow cytometry analysis in Experimental Example 5.
Fig. 8A shows graphs representing the results of flow cytometry analysis in Experimental Example 5.
Fig. 8B shows graphs representing the results of flow cytometry analysis in Experimental Example 5.

Detailed Description of the Invention

[Production method for genetically modified megakaryocyte]

In one embodiment, the present invention provides a production method for a genetically modified megakaryocyte, the method including: a step of introducing a Cas family protein and gRNA into a megakaryocyte to modify a subject gene. In the production method of the present embodiment, the Cas family protein and the gRNA to be introduced into the megakaryocyte form a complex beforehand.

In general, the amount of an intracellular protein is larger when an expression vector is introduced into a cell and expressed intracellularly than when a protein is directly introduced into a cell. Therefore, it is common technical knowledge for a person skilled in the art to introduce a Cas family protein and gRNA into a cell in the form of an expression vector to perform genome editing with high efficiency. On the other hand, as will be described later in Examples, the inventors of the present invention have elucidated that the subject gene can be efficiently modified by introducing the complex of the Cas family protein and the gRNA into the megakaryocyte to perform genome editing.

In addition, as will be described later in Examples, genetic modification can be performed at an early stage and with high efficiency by introducing the complex of the Cas family protein and the gRNA, as compared to a case in which a plasmid DNA is introduced to perform genome editing.

(Megakaryocyte)

In the production method of the present embodiment, the megakaryocyte may be derived from humans or may be derived from non-human animals as long as it is appropriately selected depending on the purpose. It may be a megakaryocyte collected from a human or non-human animal, may be a megakaryocyte obtained by inducing differentiation from hematopoietic stem cells collected from a human or non-human animal, or may be a megakaryocyte obtained by inducing differentiation of pluripotent stem cells derived from a human or non-human animal. Examples of the pluripotent stem cells include embryonic stem cells (ES cells) and induced pluripotent stem cells (iPS cells).

The megakaryocyte may be genetically engineered. For example, it may be a megakaryocyte immortalized by forcibly expressing a BMI1 gene, a c-MYC gene, and a BCL-xL gene in the presence of doxycycline. Such a megakaryocyte can be expansion-cultured in the presence of doxycycline, and removing the doxycycline makes it possible to produce a platelet.

(Cas family protein)

Examples of the Cas family protein include Cas9, Cpf1 (also known as Cas 12a), C2C1 (also known as Cas12b), C2C2 (also known as Cas13a), CasX, CasY, Cas1, Cas2, Cas3, Cas4, Cas5, Cas6, Cas7, Cas8, and Cas10. CRISPR-Cas systems are classified into class 1 and class 2. Conventionally, those in class 2 with Cas9 as a signature one have been generally used, but those in class 1 can now also be utilized.

The Cas family protein may be one in which these proteins have been modified. For example, it may be a nickase-modified nuclease in which one of two existing wild-type nuclease domains has been modified to an inactive type, or may be dCas9 in which both have been modified to an inactive type. Alternatively, it may be Cas9-HF, HiFi-Cas9, eCas9, or the like having improved target specificity. Furthermore, it may be one in which these Cas9s are fused with another protein (enzyme or the like).

Examples of Cas9 proteins include those derived from *Streptococcus pyogenes, Staphylococcus aureus*, *Streptococcus thermophilus*, and *Geobacillus stearothermophilus.* Examples of Cpf1 proteins include those derived from *Acidaminococcus, Lachnospira, Chlamydomonas*, and *Francisella novicida.*

(gRNA)

As the gRNA, one corresponding to the Cas family protein to be used is used. The gRNA determines the position on genomic DNA in which the Cas family protein induces double-stranded DNA break, that is, the subject gene to be modified.

In the present specification, the target base sequence of the gRNA refers to a base sequence on single-stranded DNA forming a complementary strand with DNA to which the spacer base sequence of the gRNA is hybridized. The spacer sequence of the gRNA complementarily binds to the antisense strand of the target sequence. Accordingly, the spacer base sequence of the gRNA and the base sequence of the sense strand of the target sequence have high sequence identity, and the spacer base sequence of the gRNA and the base sequence of the antisense strand of the target sequence are generally complementary.

The gRNA may be a complex of CRISPR RNA (crRNA) and trans-activated CRISPR RNA (tracrRNA), or may be a single gRNA (sgRNA) in which tracrRNA and crRNA are combined.

Examples of methods of preparing gRNA in the form of RNA include a method of producing a construct in which a promoter such as T7 has been added upstream of a nucleic acid fragment encoding gRNA, thereby synthesizing by an in vitro transcription reaction; and a method of chemical synthesis. When chemically synthesizing the gRNA, chemically modified RNA may be used.

For example, when a base sequence from which a PAM sequence has been removed from the target base sequence is "5'-NNNNNNNNNNNNNNNNNNNN-3'" (SEQ ID NO: 1), the base sequence of sgRNA that specifically breaks the target base sequence can be "5'-NNNNNNNNNNNNNNNNNNNNGUUUUAGAGCUAGAAAUAGCAAGUUAAAA UAAGGCUAGUCCGUUAUCAACUUGAAAAAGUGGCACCGAGUCGGUGCUUU UUUU-3'" (SEQ ID NO: 2).

As long as the sgRNA functions, the base sequence of the sgRNA may have mutation with respect to the base sequence set forth in SEQ ID NO: 2. More specifically, it may be a base sequence in which one or several bases have been deleted, substituted, or added in the base sequence set forth in SEQ ID NO: 2. Here, the one or several bases may be 1 to 10 bases, for example, 1 to 5 bases, for example, and 1 to 3 bases, for example.

When the gRNA is a complex of crRNA and tracrRNA, the base sequences of the crRNA and the tracrRNA can be the following base sequences.

First, a base sequence from which a PAM sequence has been removed from the target base sequence is used as a spacer base sequence. Subsequently, a base sequence in which a scaffold sequence has been ligated to the 3'-end of the spacer base sequence is designed and used as the base sequence of the crRNA. For example, when a base sequence (spacer base sequence) from which a PAM sequence has been removed from the target base sequence is "5'-NNNNNNNNNNNNNNNNNNNN-3'" (SEQ ID NO: 1), the base sequence of the crRNA can be "5'-NNNNNNNNNNNNNNNNNNNNGUUUUAGAGCUAUGCUGUUUUG-3'" (SEQ ID NO: 3). In addition, the base sequence of the tracrRNA can be "5'-CAAAACAGCAUAGCAAGUUAAAAUAAGGCUAGUCCGUUAUCAACUUGAAA AAGUGGCACCGAGUCGGUGC-3'" (SEQ ID NO: 4), for example.

As long as the crRNA functions, the base sequence of the crRNA may have mutation with respect to the base sequence set forth in SEQ ID NO: 3. More specifically, it may be a base sequence in which one or several bases have been deleted, substituted, or added in the base sequence set forth in SEQ ID NO: 3. Here, the one or several bases may be 1 to 10 bases, for example, 1 to 5 bases, for example, and 1 to 3 bases, for example.

In addition, as long as the tracrRNA functions, the base sequence of the tracrRNA may be a base sequence in which one or several bases have been deleted, substituted, or added in the base sequence set forth in SEQ ID NO: 4. Here, the one or several bases may be 1 to 10 bases, for example, 1 to 5 bases, for example, and 1 to 3 bases, for example.

(Introduction of Cas family protein and gRNA)

A method (transfection method) for introducing the Cas family protein and the gRNA into the megakaryocyte is not particularly limited, and examples thereof include a lipofection method, an electroporation method, and a microinjection method. In the present specification, "transfection" refers to the introduction of a nucleic acid or protein into a cell by a method other than a viral infection. When performing transfection, a commercially available transfection reagent can be appropriately used.

As the transfection reagent, a reagent capable of introducing a protein into a cell is preferable, and for example, Lipofectamine CRISPRMAX (Thermo Fisher Scientific K.K.), a Pro-DeliverIN CRISPR Transfection Reagent (OZ Biosciences), an Avalanche (R)- CRISPR Transfection Reagent (EZ Biosytems LLC.), and the like can be used.

In addition, the electroporation method can be performed using a device such as NEPA21 (Nepa Gene Co., Ltd.), Neon (Thermo Fisher Scientific K.K.), 4D-Nucleofector (Lonza Bioscience), and MaxCyte system (MaxCyte, Inc.).

The Cas family protein and the gRNA are preferably introduced into the megakaryocyte in the form of the complex. The Cas family protein and the gRNA can be formed into the complex by being mixed. Before the introduction into the megakaryocyte, the Cas family protein and the gRNA may be mixed, and incubated for several minutes (for example, 1 to 10 minutes, 1 to 7 minutes, 1 to 5 minutes, 3 to 5 minutes, or around 5 minutes), for example, incubated for about 5 minutes, thereby promoting the complex formation.

(Subject gene)

In the production method of the present embodiment, the base sequence on the subject gene is set as the target base sequence of the gRNA. In the production method of the present embodiment, the subject gene may be a combination of (i) HLA-A gene, HLA-B gene, and HLA-C gene. Alternatively, the subject gene may be (ii) B2M gene. Furthermore, "modifying the subject gene" may be disruption of the subject gene.

Here, disruption of the subject gene means that deletion or insertion is introduced at the break site of the subject gene by the complex of the Cas family protein and the gRNA, and as a result, introduction of frameshift and a termination codon, largescale deletion, and the like occur, causing non-expression of a functional protein from the subject gene.

A cell surface protein called a human leukocyte antigen (HLA) or a major histocompatibility complex (MHC) plays the most important role in distinguishing between self and non-self cells.

HLA's are classified into class 1 and class 2. HLA proteins of class 1 are expressed in most cell types in a body. The HLA proteins of class 1 have a function of forming a heterodimer with β2-Microglobulin (B2M) to be expressed on a cell surface, thereby presenting peptides to CD8-positive cytotoxic T cells to induce activation. The antigenic peptides presented are endogenous, and have a length of 8 to 10 amino acids in many cases.

Those classified into HLA class 1 are mainly six genes of an HLA-A gene, an HLA-B gene, an HLA-C gene, an HLA-E gene, an HLA-F gene, and an HLA-G gene. In addition, many pseudogenes (HLA-H, HLA-J, HLA-K, HLA-L, HLA-P, HLA-T, HLA-U, HLA-V, HLA-W, HLA-X, HLA-Y, and the like) are also known. Among these, the three genes of the HLA-A gene, HLA-B gene, and HLA-C gene have a particularly great sequence diversity among individuals, and play a major role in distinguishing between self and non-self in transplantation immunity.

HLA proteins of class 2 are mainly expressed in immune cells such as macrophages, dendritic cells, activated T cells, and B cells. The HLA proteins of class 2 have a function of forming a heterodimer by an α chain and a β chain, thereby presenting peptides to CD4 helper T cells to induce activation. The antigenic peptides presented are exogenous, and have a length of 15 to 24 amino acids in many cases.

Those classified into HLA class 2 are HLA-DR (α chain: HLA-DRA, β chain: HLA-DRB), HLA-DQ (α chain: HLA-DQA1, β chain: HLA-DQB1), HLA-DP (α chain: HLA-DPA1 or HLA-DPA2, β chain: HLA-DPB1 or HLA-DPB2). In addition, many pseudogenes (HLA-DMA, HLA-DMB, HLA-DOA, HLA-DOB) are also known to exist.

An HLA-A protein, an HLA-B protein, and an HLA-C protein are highly associated with the rejection reaction when performing cell transplantation. Therefore, by producing the genetically modified megakaryocyte in which the HLA-A gene, the HLA-B gene, and the HLA-C gene of the megakaryocyte have been disrupted, and further producing the platelet, the expression of the HLA protein on the platelet surface can be abolished. This makes it possible to expect to reduce the antigenicity at the time of platelet transplantation. In addition, a transplantable platelet preparation can be provided to patients having an antibody against HLA class 1.

The target base sequence of the gRNA for disruption of the HLA-A gene, the HLA-B gene, and the HLA-C gene of the megakaryocyte may be designed on the basis of the base sequences of the determined HLA-A gene, HLA-B gene, and HLA-C gene by determining HLA alleles of the megakaryocyte.

The determination of the HLA alleles can be performed by conventionally known methods such as a PCR-reverse Sequence Specific Oligonucleotide (PCR-rSSO) method, a PCR-Sequence specific primer (SSP) method, a PCR-Sequence based typing (SBT) method, and a next-generation sequencing method, and can be performed using a commercially available HLA typing kit or the like. In addition, HLA reporter, HLA-PRG, hla-genotyper, PHLAT, OptiType, neXtype, Athlates, HLAforest, SOAP-HLA, HLAminer, seq2HLA, GATK HLA Caller, and the like are known as software for performing HLA typing from sequence data of next-generation sequencers such as whole genome sequencing (WGS), exome sequencing (WES), and RNA-seq, and the above software may be used.

In addition, the megakaryocyte can be made deficient in the HLA-A protein, the HLA-B protein, and the HLA-C protein by deleting the B2M gene required for cell surface presentation of the HLA protein of class 1.

Furthermore, "modifying the subject gene" is not limited to disruption of the subject gene, and may be editing the base sequence of the subject gene. As a result, a modified protein is expressed from the modified subject gene.

Specifically, by specifically breaking the subject gene in the presence of donor DNA to induce double-stranded DNA break, homologous recombination can be induced in the repairing process of the double-stranded DNA break, thereby modifying the subject gene.

As the donor DNA, it is preferable to use one having the sequence identity with a region including before and after the position of double-stranded DNA break of the subject gene, and having a desired base sequence. The donor DNA may be single-stranded DNA or may be double-stranded DNA. Furthermore, the donor DNA may be DNA having a single base sequence or may be a mixture of DNA having a plurality of base sequences.

Here, the phrase "having the sequence identity" means a 90% or more base sequence match between the donor DNA and the region including the double-strand break position of the subject gene to be targeted. In the donor DNA, it is preferable that 95% or more of the base sequence match the region including the double-strand break position of the genomic DNA, and it is more preferable that 99% or more of the base sequence match the region.

The donor DNA may be single-stranded DNA of about 50 to 5,000 bases or may be double-stranded DNA of about 50 to 5,000 base pairs. When the donor DNA is single-stranded DNA, the donor DNA may have the sequence identity with any strand of the double strands of the genomic DNA.

Here, the sequence identity of a query base sequence with respect to a reference base sequence can be obtained as follows, for example. First, alignment of the reference base sequence and the query base sequence is performed. Here, each base sequence may contain a gap so as to maximize the sequence identity. Subsequently, the number of matched bases of the bases in the reference base sequence and the query base sequence can be calculated to obtain the sequence identity according to Equation (F1).

$$\text{Sequence identity }(\%) = \text{number of matched bases/total number of bases in}$$

$$\text{query base sequence} \times 100 \qquad (\text{F1})$$

[Genetically modified megakaryocyte]

In one embodiment, the present invention provides a genetically modified megakaryocyte in which (i) HLA-A, HLA-B, and HLA-C genes, or (ii) B2M gene is disrupted.

The genetically modified megakaryocyte of the present embodiment lacks the expression of the HLA protein. In addition, an HLA-deficient platelet can be produced from the megakaryocyte of the present embodiment. Therefore, the genetically modified megakaryocyte of the present embodiment makes it possible to provide the megakaryocyte or the platelet in which HLA antigen-mediated immune rejection is reduced when being transplanted, even in the case of allogeneic transplantation. Because the platelet preparation is deactivated within a few days even if it has been produced from blood donation, the medical and industrial usefulness of cells which can be inexhaustibly expansion-cultured while maintaining the platelet production ability, such as iPS cells-derived megakaryocytes, is extremely high.

In the related art, there was no choice but to produce megakaryocytes and platelets from iPS cells derived from HLA type-matched donors. On the other hand, according to the method of the present embodiment, a single HLA-deficient platelet preparation can be transplanted to a large number of recipients. As a result, the cost of the platelet preparation can be dramatically reduced as compared to autologous transplantation.

[Production method for modified platelet]

In one embodiment, the present invention provides a production method for a modified platelet, the method including: a step of obtaining a genetically modified megakaryocyte by introducing a Cas family protein and gRNA into a megakaryocyte to modify a subject gene; and a step of obtaining a modified platelet by producing a platelet from the genetically modified megakaryocyte.

The megakaryocyte, the Cas family protein, the gRNA, the subject gene, and the like are the same as those described above. The megakaryocyte may be obtained by differentiating pluripotent stem cells. The modified platelet can be produced by producing the platelet from the genetically modified megakaryocyte. Here, the modified platelet means a platelet in which the expression of a protein encoded by the subject gene, and the like have been changed, as compared to a wild-type platelet. A production method for the platelet from the megakaryocyte is not particularly limited, and a commonly performed method can be appropriately utilized.

In the production method of the present embodiment, the introduction of the Cas family protein and the gRNA into the megakaryocyte is preferably performed by the electroporation method. As will be described later in Examples, the inventors of the present invention have elucidated that the subject gene can be more efficiently modified by introducing the complex of the Cas family protein and the gRNA into the megakaryocyte to perform genome editing.

In the production method of the present embodiment, the subject gene is (i) HLA-A, HLA-B, and HLA-C genes, or (ii) B2M gene, and the above-mentioned modification may be disruption of the above-mentioned subject gene. The HLA-A gene, the HLA-B gene, the HLA-C gene, and the B2M gene are the same as those described above.

By disruption of these genes, an HLA-deficient megakaryocyte can be produced. In addition, an HLA-deficient platelet can be produced from the HLA-deficient megakaryocyte. That is, in this case, the modified platelet is the HLA-deficient platelet.

In one embodiment, the present invention provides a production method for a low-antigenic platelet, the method including the following steps: a step (a) of inducing differentiation of human-derived pluripotent stem cells into immortalized megakaryocytes; a step (b) of introducing a complex of a Cas family protein and gRNA into the immortalized megakaryocytes to modify or cause deficiency of two or more HLA class 1 proteins using a CRISPER-Cas genetic modification technique; a step (c) of proliferating the immortalized megakaryocytes in which two or more HLA class 1 proteins have been modified or made deficient; and a step (d) of producing platelets from the proliferated megakaryocytes.

[HLA-deficient platelet]

In one embodiment, the present invention provides an HLA-deficient platelet which is deficient in two or more HLA class 1 proteins. The two or more HLA class 1 proteins are preferably two or more proteins selected from the group consisting of the HLA-A protein, the HLA-B protein, and the HLA-C protein, and more preferably the HLA-A protein, the HLA-B protein, and the HLA-C protein.

In one embodiment, the present invention provides an HLA-deficient platelet which is deficient in an HLA-A protein, an HLA-B protein, and an HLA-C protein. Alternatively, the present invention can also provide the above-mentioned HLA-deficient platelet which is deficient in the HLA-A protein, the HLA-B protein, and/or the HLA-C protein. As described above, the HLA-deficient platelet of the present embodiment is deficient in the HLA proteins. Therefore, HLA antigen-mediated immune rejection can be reduced when being transplanted, even in the case of allogeneic transplantation. In addition, the HLA-deficient platelet of the present embodiment can be mass-produced by the above-mentioned method.

[Other embodiments]

In one embodiment, the present invention provides a therapeutic method for a platelet-related disorder, the method including: a step of introducing a complex of a Cas family protein and gRNA into a megakaryocyte to modify (i) HLA-A, HLA-B, and HLA-C genes, or (ii) B2M gene, thereby obtaining an HLA-deficient megakaryocyte; a step of producing a platelet from the above-mentioned HLA-deficient megakaryocyte to obtain an HLA-deficient platelet; and a step of administering an effective amount of the above-mentioned HLA-deficient platelet to a patient requiring treatment. Here, the megakaryocyte, the Cas family protein, the gRNA, the subject gene, and the like are the same as those described above.

In the therapeutic method of the present embodiment, the platelet-related disorder is a pathological condition in which severe bleeding is caused or bleeding is predicted due to a decrease in the number of platelets or an abnormality in function. Furthermore, patients may also have platelet refractoriness. In addition, the HLA-deficient platelet is preferably administered to patients by intravenous drip infusion.

Examples

Next, the present invention will be described in more detail with reference to examples, but the present invention is not limited to the following examples.

[Experimental Example 1]

(Knockout of HLA-A/B/C genes of megakaryocytes)

A Cas9 protein and sgRNA were introduced into megakaryocytes to knock out an HLA-A gene, an HLA-B gene, and an HLA-C gene (hereinafter, may be referred to as "HLA-A/B/C genes").

As the megakaryocytes, an immortalized human megakaryocyte cell line (imMKCL) which forcibly expresses a BMI1 gene, a c-MYC gene, and a BCL-xL gene in the presence of Doxycycline (Clontech Laboratories, Inc.) was used (refer to Nakamura S., et al., Expandable megakaryocyte cell lines enable clinically applicable generation of platelets from human induced pluripotent stem cells, Cell Stem Cell, 14 (4), 535-548, 2014). Expansion culture is possible in the presence of the doxycycline, and removing the doxycycline makes it possible to produce platelets.

As the Cas9 protein, a commercially available product (product name "TrueCut™ Cas9 Protein v2", catalog number "A36498", Thermo Fisher Scientific K.K.) was used.

As the sgRNA, three types of sgRNA were used, which are synthetic sgRNA targeting the HLA-A gene ("HLA-A-ex3g1", the target base sequence shown in SEQ ID NO: 5), synthetic sgRNA targeting the HLA-B gene ("HLA-B-ex2g1", the target base sequence shown in SEQ ID NO: 6), and synthetic sgRNA targeting the HLA-C gene ("HLA-C12:02-ex3g1", the target base sequence shown in SEQ ID NO: 7).

15 $\mu$g of the Cas9 protein, 1.25 $\mu$g of the HLA-A-ex3g1, 1.25 $\mu$g of the HLA-B-ex2g1, and 1.25 $\mu$g of the HLA-C12:02-ex3g1 were mixed, incubated, and thereafter introduced into $3 \times 10^5$ megakaryocytes by the electroporation method. For the electroporation, a 4D-Nucleofector™ (Lonza Bioscience) and a P4 Primary Cell 4D-Nucleofector™ X Kit S (catalog number "V4XP-4032", Lonza Bioscience) were used.

The megakaryocytes after electroporation were added to a medium to be cultured under the culture conditions of 37°C and 5% $CO_2$. The composition of the medium is shown in Table 1 below.

[Table 1]

| Component | Place of supply | Final concentration |
|---|---|---|
| Iscove's modified Dulbecco's medium (IMDM) | Sigma-Aldrich Corporation | - |
| Fetal bovine serum (FBS) | Thermo Fisher Scientific K.K. | 15% |
| Penicillin-streptomycin-glutamine | Thermo Fisher Scientific K.K. | 1% |
| Insulin-transferrin-sodium selenite solution (ITS-G) | Thermo Fisher Scientific K.K. | 1% |

(continued)

| Component | Place of supply | Final concentration |
|---|---|---|
| 1-Thioglycerol | Sigma-Aldrich Corporation | 0.45 mM |
| L-ascorbic acid | Sigma-Aldrich Corporation | 50 $\mu$g/mL |
| TPO mimic compound | Nissan Chemical Corporation | 0.2 $\mu$g/mL |
| Human Stem Cell Factor (SCF) | R&D Systems, Inc. | 50 ng/mL |
| Doxycycline | Clontech Laboratories, Inc. | 1 $\mu$g/mL |

The megakaryocytes were recovered after 3 days and after 6 days from the start of the culture to be stained with an FITC-labeled anti-HLA-A/B/C antibody (catalog number "555552", BD Biosciences). Subsequently, flow cytometry analysis was performed using FACSVerse (BD Biosciences) to measure the proportion of a cell population in which all HLA-A/B/C genes had been knocked out.

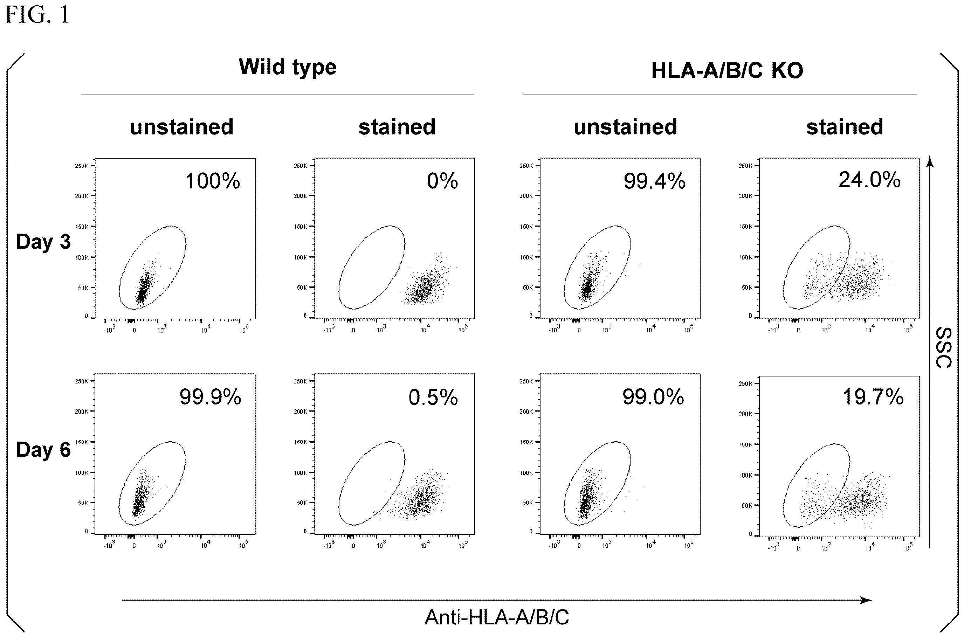

Fig. 1 shows graphs representing the results of the flow cytometry analysis. In Fig. 1, "Wild type" indicates the results of wild-type megakaryocytes, and "HLA-A/B/C KO" indicates the results of the megakaryocytes in which knockout treatment of the HLA-A gene, the HLA-B gene, and the HLA-C gene had been performed. Furthermore, "stained" indicates the results of staining with the FITC-labeled anti-HLA-A/B/C antibody, and "unstained" indicates the results of not staining. Furthermore, "Day 3" indicates the measurement results after 3 days from the start of the culture, and "Day 6" indicates the measurement results after 6 days from the start of the culture.

As a result, it became clear that in the megakaryocytes in which the knockout treatment of the HLA-A/B/C genes had been performed, 24.0% (after 3 days) and 19.7% (after 6 days) of the entire cell population became negative for the HLA-A protein, the HLA-B protein, and the HLA-C protein (hereinafter, may be referred to as "HLA-A/B/C proteins"). This result shows that genome editing could be efficiently performed on the megakaryocytes by the method of the present experimental example.

[Experimental Example 2]

(Knockout of B2M gene of megakaryocytes)

A Cas9 protein and sgRNA were introduced into megakaryocytes to knock out a B2M gene. Furthermore, knockout of the HLA-A/B/C genes was also performed for comparison. As the megakaryocytes, the same immortalized human megakaryocyte cell line (imMKCL) as in Experimental Example 1 was used.

As the Cas9 protein, a commercially available product (product name "TrueCut™ Cas9 Protein v2", catalog number "A36498", Thermo Fisher Scientific K.K.) was used.

As the sgRNA, synthetic sgRNA targeting the B2M gene ("B2M-exlg2", the target base sequence shown in SEQ ID NO: 8), synthetic sgRNA targeting the HLA-A gene ("HLA-A-ex3g1", the target base sequence shown in SEQ ID NO: 5), synthetic sgRNA targeting the HLA-B gene ("HLA-B-ex2g1", the target base sequence shown in SEQ ID NO: 6), and synthetic sgRNA targeting the HLA-C gene ("HLA-C12:02-ex3g1", the target base sequence shown in SEQ ID NO: 7) were used.

For knockout of the B2M gene, 5 $\mu$g of the Cas9 protein and 1.25 $\mu$g of the B2M-exlg2 were mixed, and introduced into $3 \times 10^5$ megakaryocytes by the electroporation method. In knockout of the HLA-A/B/C genes, 15 $\mu$g of the Cas9 protein, 1.25 $\mu$g of the HLA-A-ex3g1, 1.25 $\mu$g of the HLA-B-ex2g1, and 1.25 $\mu$g of the HLA-C12:02-ex3g1 were mixed, and introduced into $3 \times 10^5$ megakaryocytes by the electroporation method. MaxCyte STX (MaxCyte, Inc.) was used for the electroporation.

The megakaryocytes after electroporation were added to a medium having the composition shown in Table 1 above to be cultured under the culture conditions of 37°C and 5% $CO_2$. The megakaryocytes were recovered after 6 days from the start of the culture to be stained with an FITC-labeled anti-HLA-A/B/C antibody (catalog number "555552", BD Biosciences). Subsequently, flow cytometry analysis was performed using FACSVerse (BD Biosciences) to measure the proportion of a cell population in which all HLA-A/B/C genes had been knocked out.

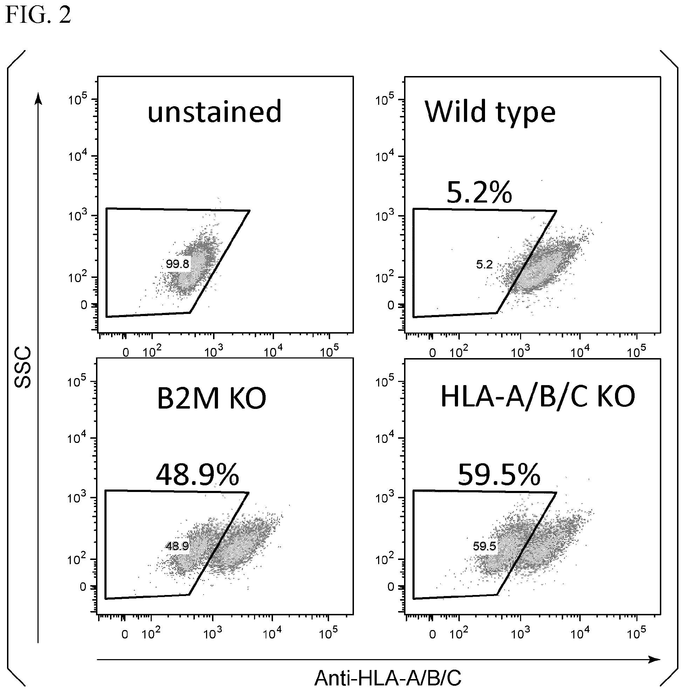

Fig. 2 shows graphs representing the results of the flow cytometry analysis. In Fig. 2, "Wild type" indicates the results of wild-type megakaryocytes, "B2M KO" indicates the results of the megakaryocytes in which knockout treatment of the B2M gene had been performed, and "HLA-A/B/C KO" indicates the results of the megakaryocytes in which knockout treatment of the HLA-A gene, the HLA-B gene, and the HLA-C gene had been performed. Furthermore, "unstained" indicates the results of not staining with the FITC-labeled anti-HLA-A/B/C antibody.

As a result, it became clear that in the megakaryocytes in which the knockout treatment of the B2M gene had been performed, 48.9% of the entire cell population became HLA-A/B/C protein-negative. In addition, it became clear that in the megakaryocytes in which the knockout treatment of the HLA-A/B/C genes had been performed, 59.5% of the entire cell population became HLA-A/B/C protein-negative. This result further supports that genome editing can be efficiently performed on the megakaryocytes by the method of the present experimental example.

[Experimental Example 3]

(Subcloning of HLA-A/B/C genes-knocked out megakaryocytes)

In order to purify the megakaryocytes in which the knockout treatment of the HLA-A/B/C genes had been performed in Experimental Example 1, a subcloning method by colony formation was performed. As a medium for subcloning, a medium having the composition shown in Table 2 below was used.

[Table 2]

| Component | Place of supply | Final concentration |
|---|---|---|
| MethoCult H4230 (catalog number "04230") | STEMCELL Technologies Inc. | - |
| TPO mimic compound | Nissan Chemical Corporation | 0.2 $\mu$g/mL |
| Human Stem Cell Factor (SCF) | R&D Systems, Inc. | 50 ng/mL |
| Doxycycline | Clontech Laboratories, Inc. | 1 $\mu$g/mL |

The megakaryocytes in which the knockout treatment of the HLA-A/B/C genes had been performed in Experimental Example 1 were added to the medium having the composition shown in Table 2 above, stirred, and seeded on a 100 mm dish. The number of seeded cells was adjusted to $5 \times 10^4$ cells/dish. Subsequently, the cells were cultured under the culture conditions of 37°C and 5% $CO_2$ for 8 to 12 days.

Subsequently, single colonies were collected under microscopic observation, and seeded in each well of a 96-well plate, in which 200 $\mu$L of the medium having the composition shown in Table 1 above was dispensed into each well, to be further cultured under the culture conditions of 37°C and 5% $CO_2$ for 3 to 7 days.

Subsequently, the proliferated megakaryocytes were recovered to be stained with an FITC-labeled anti-HLA-A/B/C antibody (catalog number "555552", BD Biosciences). Subsequently, flow cytometry analysis was performed using FACSVerse (BD Biosciences).

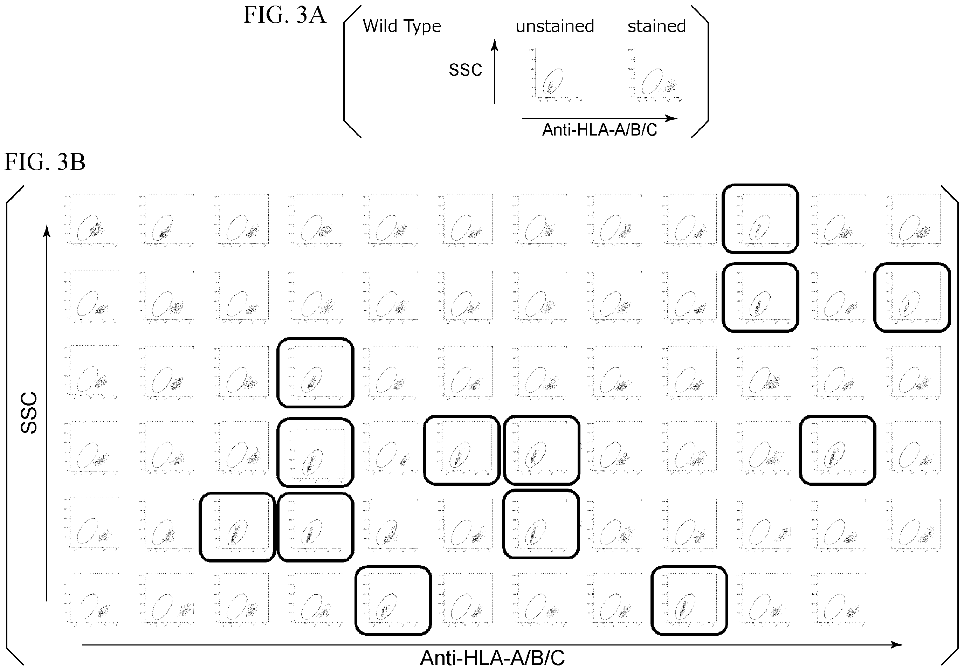

First, as shown in Fig. 3A, flow cytometry analysis of anti-HLA-A/B/C antibody unstained cells and stained cells was performed using wild-type megakaryocytes to perform gating setting that defines an HLA-A/B/C protein-negative cell population. In Fig. 3A, "stained" indicates the results of staining with the FITC-labeled anti-HLA-A/B/C antibody, and "unstained" indicates the results of not staining. In addition, ellipses in the graph indicate the gating that defines the HLA-A/B/C protein-negative cell population.

Subsequently, using the above-mentioned gating setting, the expression of the HLA-A/B/C proteins in the megakaryocytes cloned by subcloning was evaluated. Fig. 3B is a graph representing the results of flow cytometry analysis of each megakaryocyte clone. In Fig. 3B, graphs surrounded by a thick line are graphs representing HLA-A/B/C protein-negative megakaryocyte clones.

As a result, it became clear that 13 clones out of 71 clones were HLA-A/B/C protein-negative as shown in Fig. 3B. From this result, it became clear that the production of the megakaryocyte cell line required for the production of the HLA-A/B/C proteins-deficient platelet preparation is possible.

[Experimental Example 4]

(Platelet production from HLA null megakaryocytes)

In the same manner as in Experimental Example 1, HLA-A/B/C genes of an immortalized human megakaryocyte cell line (imMKCL) were knocked out, and HLA-A/B/C protein-negative megakaryocytes (hereinafter, may be referred to as "HLA null megakaryocytes") were sorted using FACSAria (BD Biosciences) to be recovered.

Subsequently, the recovered megakaryocytes were washed to be cultured in a medium not containing doxycycline, thereby resolving the forced expression of a BMI1 gene, a c-MYC gene, and a BCL-xL gene. Specifically, wild-type megakaryocytes and the HLA null megakaryocytes in a medium having the composition shown in Table 3 below were seeded in an E125 flask (catalog number "431143", Corning Incorporated) at the cell density of $1 \times 10^5$ cells/mL and in 25 mL/flask to be shaking-cultured at 100 rpm under the conditions of 37°C and 5% $CO_2$. As a result, platelet production was induced from the megakaryocytes.

[Table 3]

| Component | Place of supply | Final concentration |
|---|---|---|
| Iscove's modified Dulbecco's medium (IMDM) | Sigma-Aldrich Corporation | - |
| Fetal bovine serum (FBS) | Thermo Fisher Scientific K.K. | 15% |
| Penicillin-streptomycin-glutamine | Thermo Fisher Scientific K.K. | 1% |
| Insulin-transferrin-sodium selenite solution (ITS-G) | Thermo Fisher Scientific K.K. | 1% |
| 1-Thioglycerol | Sigma-Aldrich Corporation | 0.45 mM |
| L-ascorbic acid | Sigma-Aldrich Corporation | 50 $\mu$g/mL |
| TPO mimic compound | Nissan Chemical Corporation | 0.2 $\mu$g/mL |
| Human Stem Cell Factor (SCF) | R&D Systems, Inc. | 50 $\mu$g/mL |
| KP-457 | KAKEN PHARMACEUTICAL CO., LTD. | 15 $\mu$M |
| Y-27632 | FUJIFTLM Wako Pure Chemical Corporation | 10 $\mu$M |
| StemRegenin-1 (SR-1) | STEMCELL Technologies Inc. | 0.75 $\mu$M |

After culturing for 6 days, a part of the cell suspension was taken to be stained for 30 minutes with an APC-labeled anti-CD41 antibody (catalog number "303710", BioLegend, Inc.), a PE-labeled anti-CD42b antibody (catalog number "303906", BioLegend, Inc.), and an FITC-labeled anti-HLA-A/B/C antibody (catalog number "555552", BD Biosciences).

Subsequently, flow cytometry analysis was performed using FACSVerse (BD Biosciences) to examine the expression of the HLA-A/B/C proteins of the platelets (CD41/CD42b-double positive cells).

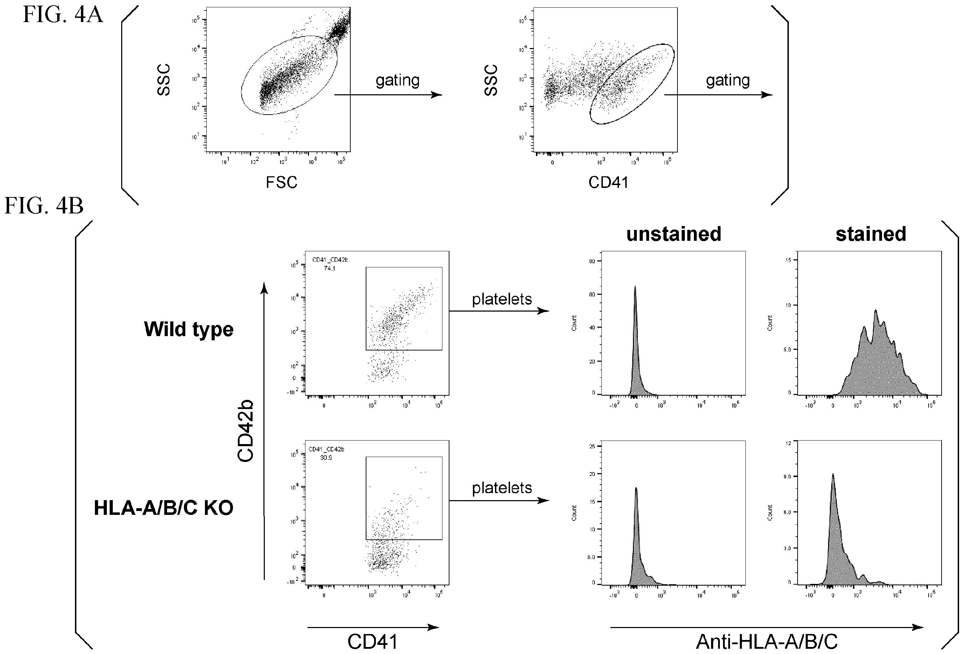

Figs. 4A and 4B are graphs representing the results of flow cytometry analysis. As shown in Fig. 4A, first, a microcell population was gated on the basis of a forwardscattered light signal (FSC)/side-scattered light signal (SSC), and the CD41-positive fraction was further gated.

Subsequently, as shown in Fig. 4B, among the gated fractions, the CD42b-positive fraction was defined as the platelets to examine the expression of the HLA-A/B/C proteins. In Fig. 4B, "Wild type" indicates the results of the wild-type megakaryocyte-derived platelets, and "HLA-A/B/C KO" indicates the results of the HLA null megakaryocyte-derived platelets. Furthermore, "stained" indicates the results of staining with the FITC-labeled anti-HLA-A/B/C antibody, and "unstained" indicates the results of not staining.

As a result, it became clear that the expression of the HLA-A/B/C proteins was positive in the wild-type megakaryocyte-derived platelets, whereas the expression of the HLA-A/B/C proteins was negative in the HLA null megakaryocyte-derived platelets.

[Experimental Example 5]

(Examination of genome editing efficiency of megakaryocytes)

A Cas9 protein and gRNA were introduced into megakaryocytes by various methods to knock out a B2M gene, and the genome editing efficiency was examined. As the megakaryocytes, the same immortalized human megakaryocyte cell line (imMKCL) as in Experimental Example 1 was used.

Table 4 below shows the conditions for introducing the Cas9 protein and gRNA examined. The Cas9 protein was used in the form of a Cas9 expression plasmid or in the form of a Cas9 protein. As the Cas9 expression plasmid, pHL-EF1a-SphcCas9-iC-A was used. pHL-FF1a-SphcCas9-iC-A was produced by cloning SphcCas9 cDNA of pHL-EF1a-SphcCas9-iP-A, which is disclosed in Li H. L., et al., Precise Correction of the Dystrophin Gene in Duchenne Muscular Dystrophy Patient Induced Pluripotent Stem Cells by TALEN and CRISPR-Cas9, Stem Cell Reports, 4, 143-154, 2015., into a pHL-EF1a-GW-iC-A vector.

As the Cas9 protein, a commercially available product (product name "TrueCut™ Cas9 Protein v2", catalog number "A36498", Thermo Fisher Scientific K.K.) was used.

The gRNA was used in the form of an expression plasmid of gRNA targeting the B2M gene or in the form of sgRNA. As the expression plasmid of gRNA targeting the B2M gene, pHL-H1-B2M-sgRNA-mFF1a-RiH (refer to Suzuki D., et al., iPSC-Derived Platelets Depleted of HLA Class I Are Inert to Anti-HLA Class I and Natural Killer Cell Immunity, Stem Cell Reports, 14, 49-59, 2020) was used.

As the sgRNA, synthetic sgRNA targeting the B2M gene ("B2M-exlg2", the target base sequence shown in SEQ ID NO: 8) was used.

In addition, the case in which the Cas9 protein and the gRNA were incubated for 5 minutes to form a complex before being introduced into the cells, and the case in which the complex was not formed were examined.

As a method for introducing the Cas9 protein and the gRNA, a lipofection method and an electroporation method were considered. For the lipofection method, Lipofectamine 2000 (Thermo Fisher Scientific K.K.), and Lipofectamine CRISPRMAX (Thermo Fisher Scientific K.K.) suitable for protein introduction were used. For the electroporation, a 4D-Nucleofector™ (Lonza Bioscience) and an Amaxa™ P4 Primary Cell 4D-Nucleofector™ X Kit S (Lonza Bioscience) were used.

[Table 4]

| Group | 1 | 2 | 3 | 4 | 5 | 6 | 7 | 8 | 9 | 10 |
|---|---|---|---|---|---|---|---|---|---|---|
| Introduction method | None | Lipofectamine 2000 | | | Lipofectamine CRISPRMAX | | | 4D-Nucleofector | | |
| Cas9 | None | Cas9 expression plasmid (1 $\mu$g) | Cas9 protein (30 pmol) | Cas9 protein (30 pmol) | Cas9 expression plasmid (1 $\mu$g) | Cas9 protein (30 pmol) | Cas9 protein (30 pmol) | Cas9 expression plasmid (1 $\mu$g) | Cas9 protein (30 pmol) | Cas9 protein (30 pmol) |
| Guide RNA | None | B2M gRNA expression plasmid (1 $\mu$g) | B2M target sgRNA (30 pmol) | B2M target sgRNA (30 pmol) | B2M gRNA expression plasmid (1 $\mu$g) | B2M target sgRNA (30 pmol) | B2M target sgRNA (30 pmol) | B2M gRNA expression plasmid (1 $\mu$g) | B2M target sgRNA (30 pmol) | B2M target sgRNA (30 pmol) |
| Introduction pretreatment | None | None | None | Incubation for 5 minutes | None | None | Incubation for 5 minutes | None | None | Incubation for 5 minutes |

The megakaryocytes after introduction of the Cas9 protein and the gRNA were cultured in the medium having the composition shown in Table 1 above under the culture conditions of 37°C and 5% $CO_2$.

After 4 days and after 7 days from the introduction of the Cas9 protein and the gRNA, each megakaryocyte was stained with an FITC-labeled anti-HLA-A/B/C antibody (catalog number "555552", BD Biosciences). Furthermore, the same cells were stained with Propidium Iodide (PI). Subsequently, flow cytometry analysis was performed using FACSVerse (BD Biosciences) to evaluate the proportion of the megakaryocytes in which the B2M gene had been knocked out. In addition, by analyzing the proportion of PI-positive cells, dead cells were detected to also evaluate the damage to the cells.

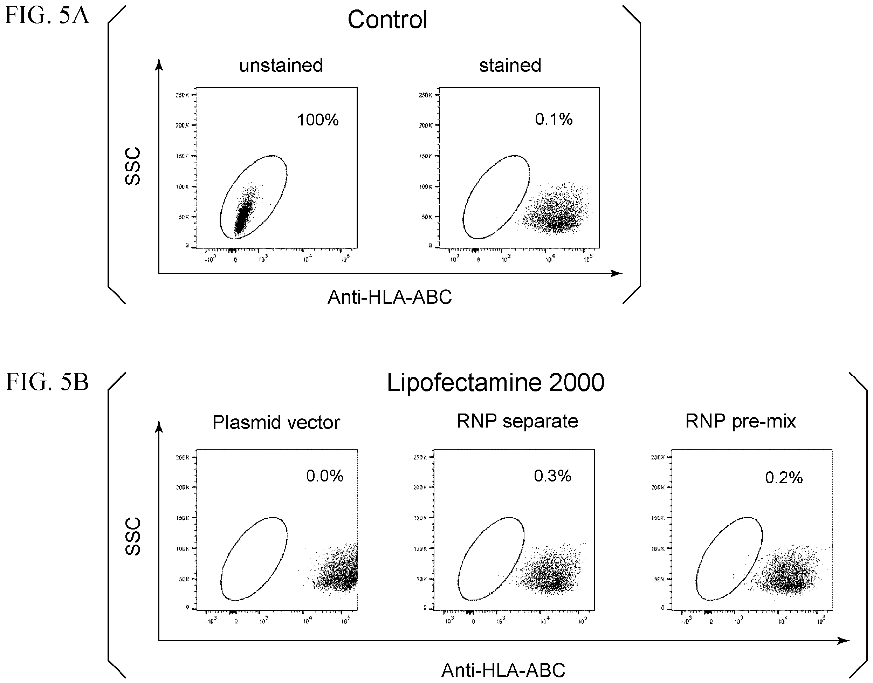

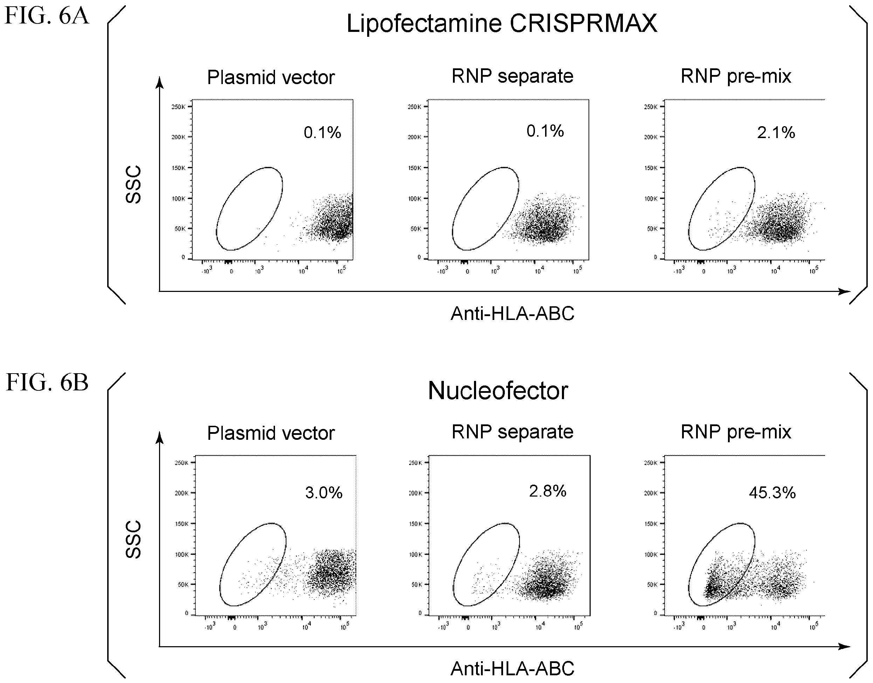

Figs. 5A and 5B, and Figs. 6A and 6B are graphs representing the analysis results after 4 days from the introduction of the Cas9 protein and the gRNA.

Fig. 5A shows the results of a control group into which the Cas9 protein and the gRNA were not introduced. In Fig. 5A, "stained" indicates the results of staining with the FITC-labeled anti-HLA-A/B/C antibody, and "unstained" indicates the results of not staining.

Fig. 5B shows the results of a group into which the Cas9 protein and the gRNA were introduced using the Lipofectamine 2000. In Fig. 5B, "Plasmid vector" indicates the results of introducing the Cas9 protein and the gRNA in the form of an expression plasmid, "RNP separate" indicates the results of not forming the complex of the Cas9 protein and the gRNA, and "RNP pre-mix" indicates the results of mixing the Cas9 protein and the gRNA and incubating thereafter to form the complex.

In Figs. 5A and 5B, "SSC" indicates a side-scattered light signal, and "Anti-HLA-ABC" indicates the staining intensity with the FITC-labeled anti-HLA-A/B/C antibody.

Fig. 6A shows the results of a group into which the Cas9 protein and the gRNA were introduced using Lipofectamine CRISPRMAX, and Fig. 6B shows the results of a group into which the Cas9 protein and the gRNA were introduced by the electroporation method.

In Figs. 6A and 6B, "Plasmid vector" indicates the results of introducing the Cas9 protein and the gRNA in the form of an expression plasmid, "RNP separate" indicates the results of not forming the complex of the Cas9 protein and the gRNA, and "RNP pre-mix" indicates the results of mixing the Cas9 protein and the gRNA and incubating thereafter to form the complex. In addition, "SSC" indicates a side-scattered light signal, and "Anti-HLA-ABC" indicates the staining intensity with the FITC-labeled anti-HLA-A/B/C antibody.

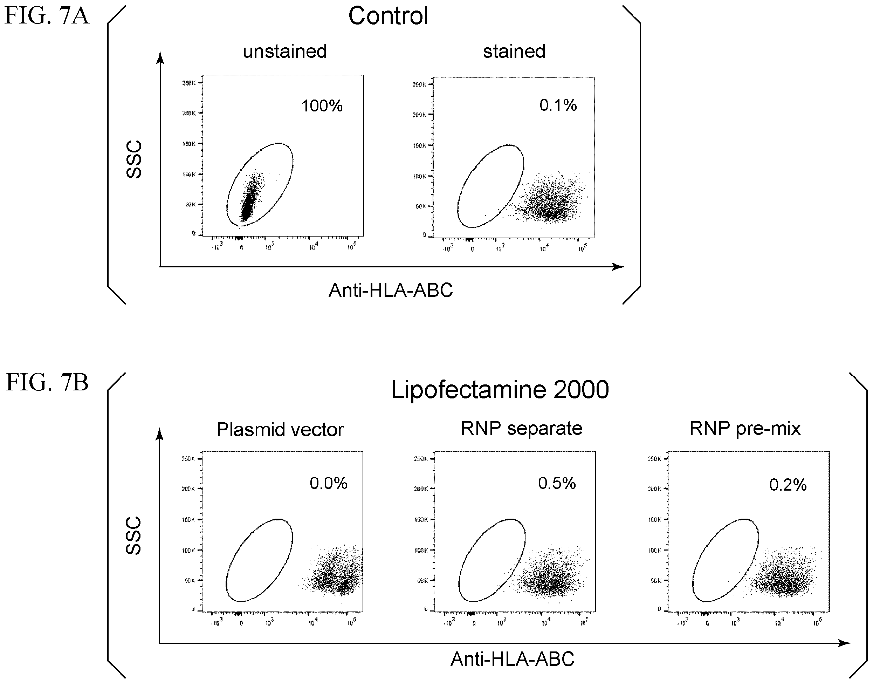

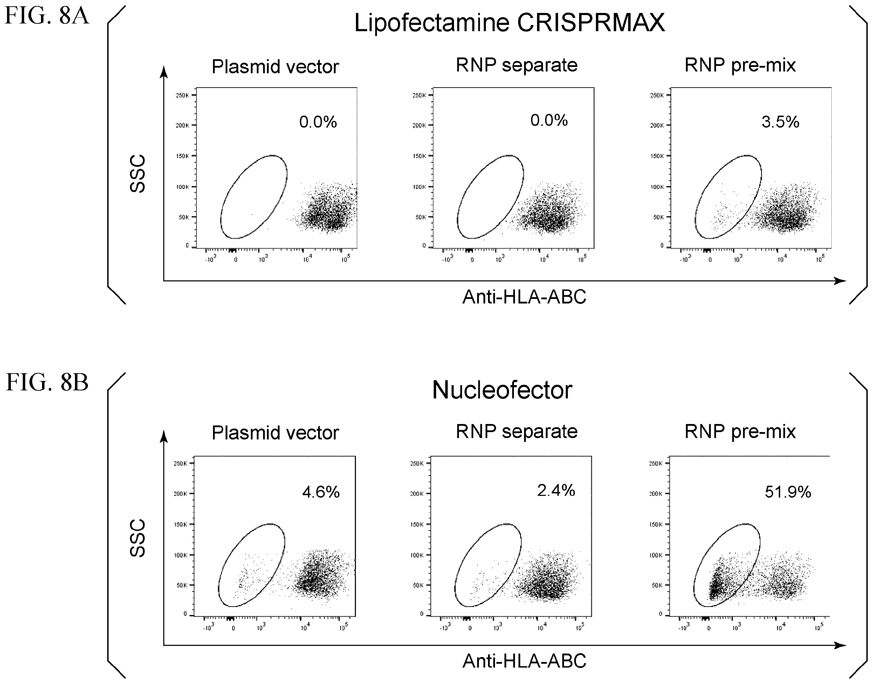

Figs. 7A and 7B, and Figs. 8A and 8B are graphs representing the analysis results after 7 days from the introduction of the Cas9 protein and the gRNA.

Fig. 7A shows the results of a control group into which the Cas9 protein and the gRNA were not introduced. In Fig. 7A, "stained" indicates the results of staining with the FITC-labeled anti-HLA-A/B/C antibody, and "unstained" indicates the results of not staining.

Fig. 7B shows the results of a group into which the Cas9 protein and the gRNA were introduced using the Lipofectamine 2000. In Fig. 7B, "Plasmid vector" indicates the results of introducing the Cas9 protein and the gRNA in the form of an expression plasmid, "RNP separate" indicates the results of not forming the complex of the Cas9 protein and the gRNA, and "RNP pre-mix" indicates the results of mixing the Cas9 protein and the gRNA and incubating thereafter to form the complex.

In Figs. 7A and 7B, "SSC" indicates a side-scattered light signal, and "Anti-HLA-ABC" indicates the staining intensity with the FITC-labeled anti-HLA-A/B/C antibody.

Fig. 8A shows the results of a group into which the Cas9 protein and the gRNA were introduced using Lipofectamine CRISPRMAX, and Fig. 8B shows the results of a group into which the Cas9 protein and the gRNA were introduced by the electroporation method.

In Figs. 8A and 8B, "Plasmid vector" indicates the results of introducing the Cas9 protein and the gRNA in the form of an expression plasmid, "RNP separate" indicates the results of not forming the complex of the Cas9 protein and the gRNA, and "RNP pre-mix" indicates the results of mixing the Cas9 protein and the gRNA and incubating thereafter to form the complex. In addition, "SSC" indicates a side-scattered light signal, and "Anti-HLA-ABC" indicates the staining intensity with the FITC-labeled anti-HLA-A/B/C antibody.

As a result, first, in the analysis using the expression plasmid vector of the Cas9 and the gRNA, genome editing was almost not recognized by the lipofection method (0.0% or 0.1%, "Plasmid vector" of Fig. 5B, Fig. 6A, Fig. 7B, and Fig. 8A), but it was slightly recognized by the electroporation method (3.0% (Fig. 6B) and 4.6% (Fig. 8B), "Plasmid vector"). This reconfirmed the difficulty of introducing the gene (plasmid DNA) into the megakaryocytes.

Next, in the analysis using the Cas9 protein and the gRNA, regardless of with or without incubation, the genome editing efficiency was very low when Lipofectamine 2000 was used (0.5% or less in all the cases, "RNP separate" and "RNP pre-mix" of Fig. 5B and Fig. 7B). This result was thought to be because Lipofectamine 2000 was not a suitable reagent for introducing the Cas9 protein or the sgRNA.

On the other hand, when the lipofection reagent for protein introduction (Lipofectamine CRISPRMAX) was used, the genome editing efficiency was 0.1% (after 4 days) and 0.0% (after 7 days) without incubation, whereas it increased to 2.1% (after 4 days) and 3.5% (after 7 days) with incubation (Fig. 6A and Fig. 8A). Furthermore, in the case of introduction by the electroporation method, depending on with or without incubation, the genome editing efficiency significantly increased from 2.8% (without incubation) to 45.3% (with incubation) after 4 days (Fig. 6B), and from 2.4% (without incubation) to 51.9% (with incubation) after 7 days (Fig. 8B).

Therefore, it became clear that when the Cas family protein and the gRNA are introduced into the megakaryocytes to perform genome editing, if the Cas family protein and the gRNA are introduced after being formed into the complex, the genome editing efficiency significantly increases (increases by double digits) in both the lipofection method suitable for protein introduction and the electroporation method. In particular, the genome editing efficiency obtained when the above-mentioned complex is introduced using the electroporation method is extremely high, and no example has been reported in which genome editing was performed on the megakaryocytes with such high efficiency.

According to the analysis results of PI staining, when the Cas9 protein and the gRNA were introduced by any of the lipofection method or the electroporation method, the proportion of the cells stained with PI was similarly low (4.3% or less after 4 days, and 1.5% or less after 7 days) regardless of with or without formation of the complex. Therefore, it became clear that in the method for introducing the complex of the Cas family protein and the gRNA into the megakaryocytes to perform genome editing, damage (cytotoxicity) to the megakaryocytes is also sufficiently low.

Industrial Applicability

According to the present invention, a technique for producing a genetically modified megakaryocyte and a modified platelet can be provided.

**Claims**

1. A production method for a genetically modified megakaryocyte, the method comprising:

   a step of introducing a CRISPR-associated (Cas) family protein and guide RNA (gRNA) into a megakaryocyte to modify a subject gene,
   wherein the Cas family protein and the gRNA to be introduced into the megakaryocyte in the step form a complex beforehand.

2. The production method according to claim 1, wherein the introduction of the Cas family protein and the gRNA is performed by a lipofection method which is suitable for protein introduction or an electroporation method.

3. The production method according to claim 1 or 2, wherein the introduction of the Cas family protein and the gRNA is performed by an electroporation method.

4. The production method according to any one of claims 1 to 3,

   wherein the subject gene is (i) Human Leukocyte Antigen (HLA)-A, HLA-B, and HLA-C genes, or (ii) β2-Microglobulin (B2M) gene, and
   the modification is disruption of the subject gene.

5. The production method according to any one of claims 1 to 4, wherein the megakaryocyte is obtained by differentiating pluripotent stem cells.

6. A genetically modified megakaryocyte in which (i) HLA-A, HLA-B, and HLA-C genes, or (ii) B2M gene is disrupted.

7. A production method for a modified platelet, the method comprising:

   a step of obtaining a genetically modified megakaryocyte by introducing a Cas family protein and gRNA into a megakaryocyte to modify a subject gene; and
   a step of obtaining a modified platelet by producing a platelet from the genetically modified megakaryocyte,
   wherein the Cas family protein and the gRNA to be introduced into the megakaryocyte in the step of obtaining a genetically modified megakaryocyte form a complex beforehand.

8. The production method according to claim 7, wherein the introduction of the Cas family protein and the gRNA is performed by a lipofection method which is suitable for protein introduction or an electroporation method.

9. The production method according to claim 7 or 8, wherein the introduction of the Cas family protein and the gRNA is performed by an electroporation method.

10. The production method according to any one of claims 7 to 9,

wherein the subject gene is (i) HLA-A, HLA-B, and HLA-C genes, or (ii) B2M gene, and
the modification is disruption of the subject gene.

11. The production method according to any one of claims 7 to 10, wherein the megakaryocyte is obtained by differentiating pluripotent stem cells.

12. A production method for a genetically modified megakaryocyte, the method comprising:

a step of introducing a Cas family protein and gRNA into a megakaryocyte to modify a subject gene,
wherein the introduction of the Cas family protein and the gRNA in the step is performed by an electroporation method.

FIG. 1

Wild type

HLA-A/B/C KO

unstained | stained | unstained | stained

Day 3

100% | 0% | 99.4% | 24.0%

Day 6

99.9% | 0.5% | 99.0% | 19.7%

Anti-HLA-A/B/C

SSC

EP 4 112 720 A1

FIG. 2

FIG. 3A

Wild Type    unstained    stained

SSC

Anti-HLA-A/B/C

FIG. 3B

SSC

Anti-HLA-A/B/C

EP 4 112 720 A1

FIG. 4A

FIG. 4B

FIG. 5A

Control

FIG. 5B

Lipofectamine 2000

FIG. 6A

## Lipofectamine CRISPRMAX

Plasmid vector — 0.1%

RNP separate — 0.1%

RNP pre-mix — 2.1%

SSC

Anti-HLA-ABC

FIG. 6B

## Nucleofector

Plasmid vector — 3.0%

RNP separate — 2.8%

RNP pre-mix — 45.3%

SSC

Anti-HLA-ABC

FIG. 7A

## Control

unstained

stained

100%

0.1%

SSC

Anti-HLA-ABC

FIG. 7B

## Lipofectamine 2000

Plasmid vector

RNP separate

RNP pre-mix

0.0%

0.5%

0.2%

SSC

Anti-HLA-ABC

FIG. 8A

## Lipofectamine CRISPRMAX

Plasmid vector — 0.0%  
RNP separate — 0.0%  
RNP pre-mix — 3.5%

SSC (y-axis) / Anti-HLA-ABC (x-axis)

FIG. 8B

## Nucleofector

Plasmid vector — 4.6%  
RNP separate — 2.4%  
RNP pre-mix — 51.9%

SSC (y-axis) / Anti-HLA-ABC (x-axis)

## INTERNATIONAL SEARCH REPORT

| International application No. |
|---|
| PCT/JP2021/007669 |

A. CLASSIFICATION OF SUBJECT MATTER
C12N 5/10(2006.01)i; A61K 35/19(2015.01)i; A61P 7/04(2006.01)i; C12N
5/078(2010.01)i; C12N 15/09(2006.01)i
FI: C12N5/10 ZNA; A61K35/19 Z; A61P7/04; C12N5/078; C12N15/09 110
According to International Patent Classification (IPC) or to both national classification and IPC

B. FIELDS SEARCHED

Minimum documentation searched (classification system followed by classification symbols)
C12N5/10; A61K35/19; A61P7/04; C12N5/078; C12N15/09

Documentation searched other than minimum documentation to the extent that such documents are included in the fields searched
| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS
(STN)

C. DOCUMENTS CONSIDERED TO BE RELEVANT

| Category* | Citation of document, with indication, where appropriate, of the relevant passages | Relevant to claim No. |
|---|---|---|
| Y | SUZUKI, D. et al., "iPSC-derived platelets depleted of HLA Class I are inert to anti-HLA class I and natural killer cell immunity", Stem Cell Reports, January 2020, vol. 14, pp. 49-59, Summary, Experimental procedures | 1-12 |
| Y | ZURIS, J. A. et al., "Cationic lipid-mediated delivery of proteins enables efficient protein-based genome editing in vitro and in vivo", nature biotechnology, 2015, vol. 33, pp. 73-80, abstract, fig. 5, page 78 | 1-12 |
| Y | KIM, S. et al., "Highly efficient RNA-guided genome editing in human cells via delivery of purified Cas9 ribonucleoproteins", Genome Research, 2014, vol. 24, pp. 1012-1019, results, pp. 1012, 1016 | 1-12 |

☐ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 April 2021 (13.04.2021) | 18 May 2021 (18.05.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020034255 A

**Non-patent literature cited in the description**

- **ISAKARI Y. et al.** Efficient gene expression in megakaryocytic cell line using nucleofection. *Int J Pharm.,* 2007, vol. 338 (1-2), 157-64
- **SEO H. et al.** A betal-tubulin-based megakaryocyte maturation reporter system identifies novel drugs that promote platelet production. *Blood Adv.,* 2018, vol. 2 (17), 2262-2272
- **KAHR W.H. et al.** Loss of the Arp2/3 complex component ARPC1B causes platelet abnormalities and predisposes to inflammatory disease. *Nat Commun.,* 2017, vol. 8, 14816
- **NAKAMURA S. et al.** Expandable megakaryocyte cell lines enable clinically applicable generation of platelets from human induced pluripotent stem cells. *Cell Stem Cell,* 2014, vol. 14 (4), 535-548
- **LI H. L. et al.** Precise Correction of the Dystrophin Gene in Duchenne Muscular Dystrophy Patient Induced Pluripotent Stem Cells by TALEN and CRISPR-Cas9. *Stem Cell Reports,* 2015, vol. 4, 143-154
- **SUZUKI D. et al.** iPSC-Derived Platelets Depleted of HLA Class I Are Inert to Anti-HLA Class I and Natural Killer Cell Immunity. *Stem Cell Reports,* 2020, vol. 14, 49-59